# EUROPEAN PATENT APPLICATION

(11) **EP 4 850 647 A2**
(43) Date of publication of application: **09.12.2026**
(21) Application number: 26204619.6
(22) Date of filing: 30.12.2023
(51) Int. Cl.: A61F 13/08

(54) **COMPRESSION GARMENT COMPRISING VISCOSE**

(62) Document number(s) of the earlier application(s) in accordance with Art. 76 EPC: 23833506.1 / 4 669 287
(71) Applicant: Essity Hygiene and Health Aktiebolag, 405 03 Göteborg (SE)
(72) Inventor: CLEMENDOT, Olivier, 405 03 Goteborg (SE)
(74) Representative: Nederlandsch Octrooibureau

(57) **Abstract**

The present invention relates to compression garments comprising a knitted fabric, wherein the knitted fabric comprises a first yarn comprising a first viscose and a second yarn comprising a second viscose. Furthermore, the invention relates to a method for the manufacture of such a compression garment.

## Description

### TECHNICAL FIELD

The present disclosure relates to a sustainable compression garment comprising a knitted fabric for use in the treatment of e.g. phlebologic diseases.

### BACKGROUND

Garments which are able to apply pressure to a body part of a subject are known as compression garments and have been used for a variety of therapeutic and non-therapeutic applications, such as treating phlebologic or lymphologic diseases. The application of pressure to the affected body part can alleviate symptoms of disease and prevent or slow disease progression. Moreover, it is thought to improve recovery after physical training.

For an efficient therapy, it is crucial that medical compression garments apply the therapeutically accurate pressure and that the patients consistently wear the garments. The garments, therefore, do not only have to be knitted precisely according to the desired compression profile, they also have to have a pleasant feel on the skin and good fit. Moreover, the donning and doffing of the garments should be easy also for patients with reduced mobility.

In addition to these technical requirements, it becomes more and more important to use sustainable materials to reduce the environmental footprint. Most of the present medical compression garments are manufactured with artificial fibers. Some newer sustainable garments make use of cotton or wool fibers. These, however, have more problems to fulfil the technical requirements of a medical compression garment.

There is, thus, a need in the art for garments which are able to apply the medically correct compression and are more sustainable than prior art garments.

### DESCRIPTION

This problem is solved by a compression garment as described in the appended claims. According to the invention, two different types of viscose yarns are used in the compression garments. These viscose yarns have very good technical properties and a low environmental impact. In a first aspect, the invention, thus, relates to a compression garment comprising a knitted fabric, wherein the knitted fabric comprises a first yarn comprising a first viscose and a second yarn comprising a second viscose.

It has been advantageously found that the combination of two different viscose yarns leads to compression garments with good technical properties as well as considerably improved sustainability. Technically, the garments have a good breathability, good thermal regulation and are unfavourable for bacterial growth. Furthermore, the materials are gentle and smooth on the skin of the wearer. The aforementioned advantageous technical properties are particularly good when one of the viscose yarns is a lyocell yarn. In certain preferred embodiments, the first viscose is, thus, lyocell.

The compression garment comprises or consists of a knitted fabric, in particular a weft-knitted fabric. The fabric can be a flat-knitted fabric or a circular-knitted fabric, wherein circular-knitted fabrics are preferred. Thus, the knitted fabric can e.g. be a circular weft knitted fabric. Accordingly, the garment can be a flat-knitted or a circular-knitted garment, wherein circular-knitted garments are preferred.

The knitted fabric comprises a first yarn comprising a first viscose and a second yarn comprising a second viscose. In certain embodiments, the knitted fabric consists of the first yarn and the second yarn. It will, however, be understood, that further yarns may be comprised in the knitted fabric. In particular, certain functional parts, such as the heel and the toe parts in the case of a stocking or pantyhose, may be knitted with other yarns. It is preferred that other yarns comprised in the garment are also sustainable yarns, such as yarns which are cellulose-based, e.g. further viscose yarns.

The knitted fabric is, thus, knitted with at least two different types of yarns. Specifically, the knitted fabric is knitted with two yarns that comprise different types of viscose. The first yarn comprises a first viscose and the second yarn comprises a second viscose. "First viscose" is used synonymously herein with "first type of viscose". "Second viscose" is used synonymously herein with "second type of viscose".

In line with its common meaning in the art, a "viscose" is a cellulose-based fiber. Viscose is also classified as a regenerated cellulose. It can be manufactured from natural sources, such as wood, in particular from wood pulp. Viscose is also called rayon. Various methods for the production of viscose are known in the art. For example, the viscose can be manufactured by the so-called viscose process or the lyocell process.

As outlined above, the first viscose can be lyocell. Viscose manufactured by the lyocell process is known as lyocell. The lyocell process is more sustainable than the viscose process, in particular because it does not require toxic carbon sulfide. Instead, the lyocell process dissolves cellulose in NMMO (N-methyl morpholine N-oxide). Lyocell is available on the market, e.g. under the trade name Tencel^{®} (Lenzing AG).

Other viscose types known in the art are also suitable for use in the invention. Preferred are viscose types which have a low environmental impact. The viscose, e.g. the second viscose, can e.g. be a viscose manufactured from cellulose derived from sustainable sources, such as sustainable forestry (e.g. FSC^{®} certified cellulose (FSC: Forest Stewardship Council)). Viscose manufactured from cellulose derived from sustainable forestry is e.g. ENKA^{®} viscose (available from ENKA GmbH & Co. KG). The second viscose can be a viscose manufactured with the viscose process.

The garment can e.g. comprise 20% by weight or more of the first viscose, such as 25% by weight or more, 30% by weight or more, 35% by weight or more, 40% by weight or more, or 45% by weight or more. For example, the garment can comprise 40% or more of the first viscose. In other words, the garment can e.g. comprise from 20% by weight to 90% by weight of the first viscose, such as from 25% by weight to 90% by weight, from 30% by weight to to 90% by weight, from 35% by weight to 90% by weight, from 40% by weight to 90% by weight, or from 45% by weight to 90% by weight.

The garment can e.g. comprise 2 % by weight or more of the second viscose, such as 3% by weight or more, or 4% by weight or more, such as approximately 5% by weight. In other words, the garment can e.g. comprise from 2% by weight to 30% by weight of the second viscose, such as from 3% by weight to 30% by weight, from 4% by weight to 30% by weight, from 2% by weight to 20% by weight, from 3% by weight to 20% by weight, from 4% by weight to 20% by weight, from 2% by weight to 10% by weight, from 3% by weight to 10% by weight, or from 4% by weight to 10% by weight.

In some embodiments, the garment comprises 35% by weight or more of the first viscose and 2% by weight or more of the second viscose. For example, the garment can comprise 40% or more of the first viscose and 3% by weight or more of the second viscose.

Further types of yarns can be used in addition to the first yarn and the second yarn. It is, however, also possible that the knitted fabric consists of the first yarn and the second yarn. If more yarns are used, they can be e.g. knitted in the heel and toe regions of a garment that is a stocking or other functional regions of the garment. Alternatively or additionally, further yarns may be used in the basic fabric.

While the term "viscose" refers to the viscose as such, it will be understood that the term "viscose yarns" includes all viscose yarns comprised by the garment, namely all yarns that comprise or consist of viscose, such as the first yarn and the second yarn. "Viscose yarns" may, thus, comprise further materials in addition to the viscose. For example, they can be a covered core yarn, wherein either the core and/or a cover yarn and/or all cover yarns are viscose. Alternatively, the viscose yarn may be an uncovered monofilament yarn consisting of viscose. Further alternatively, the viscose yarn may be a multifilament yarn in which a part or all of the filaments consist of viscose.

Preferably, the garment of the invention comprises 20% by weight or more of viscose yarns, 30% by weight or more, 40 % by weight or more, 45 % by weight or more, or 50% by weight or more. In other words, the garment can comprise from 20 % by weight to 100 % by weight, from 30% by weight to 100% by weight, from 40% by weight to 100% by weight, from 45 % by weight to 100% by weight, or from 50% by weight to 100% by weight viscose yarns, such as e.g. from 80% by weight to 100% by weight or from 90% by weight to 100% by weight.

Even though the viscose yarns may comprise further materials, it is preferred that the overall content of viscose in the garment is rather high. For example, the garment of the invention can comprise 20% by weight or more viscose, 30% by weight or more viscose, 40% by weight or more viscose, 45% by weight or more viscose, or 50% by weight or more viscose. For example, the garment of the invention can comprise 45% by weight or more viscose. In other words, the garment of the invention can comprise from 20% by weight to 90% by weight, from 30% by weight to 90% by weight, from 40% by weight to 90% by weight, from 45% by weight to 90% by weight, or from 50% by weight to 90% by weight viscose.

The viscose yarns of the invention can be used in the knitted fabric as knitting yarns (i.e. mesh-forming yarns; also known as "body yarn") and/or inlay yarns. For example, the first yarn can be a knitting yarn. In one embodiment, the first yarn is a lyocell yarn and a knitting yarn. The second yarn can be an inlay yarn. An inlay yarn normally assumes a relatively straight configuration in the knitted fabric and does not form meshes (compare "Knitting technology - a comprehensive handbook and practical guide", 3rd edition, DJ Spencer, Chapter 6.8.1). The inlay can be tucked to be fastened in the fabric. It has been surprisingly found that viscose yarns are suitable for use as knitting yarns as well as inlay yarns allowing for an overall highly sustainable stocking. Moreover, using viscose yarns as inlay yarn as well as knitting yarn also allows for a sustainable dyeing process to color the garment. The viscose can be dyed at lower temperatures and with less water than conventionally used artificial fibers.

The compression garment can comprise a tubular section. The tubular section can be adapted to cover a portion of an arm or a leg, e.g. the calf and/or the thigh, of a wearer.

The knitting yarn can be used in every course (knitted row) of the tubular section, for example in the calf area of a stocking. Similarly, the inlay yarn can be used in every course of the tubular section, for example in every course of the portion adapted to cover the calf area of a wearer.

A "course" as mentioned herein and in line with the standard meaning in the art is a substantially horizontal row of knitted loops (in an upright fabric as knitted) produced by adjacent needles (compare "Knitting technology - a comprehensive handbook and practical guide", 3rd edition, DJ Spencer, Chapter 3.3). The terms "course" and "knitted row" are used interchangeably herein.

As outlined elsewhere herein, the yarns can have different structures. The yarn can e.g. be a covered core yarn, an uncovered monofilament yarn or a multifilament yarn. In certain embodiments, the first yarn and/or the second yarn is a covered core yarn. For example, the first as well as the second yarn can be covered core yarns. Such covered core yarns comprise a core (i.e. a yarn forming the core) and one or more cover yarns (i.e. yarns forming the cover). The cover yarn or yarns are wound around the core. The covered core yarn may, e.g. be a double-covered core yarn. This is a yarn having a core and two cover yarns. The two cover yarns are wound around the core successively, thus, forming two cover layers. The cover yarns may be wound around the core in opposite directions (clock-wise and counter-clock-wise).

The first yarn and/or the second yarn may be covered core yarns and have an elastane core. In other words, the core of the first and/or second yarn may be formed of elastane. In certain embodiments, the first as well as the second yarn have an elastane core. This has the advantage that the resulting fabric provides a good elasticity which is advantageous for a medical compression garment, in particular for its donning and doffing properties, but also for the compression exerted by the garment.

The first yarn and/or the second yarn may be covered core yarns and have viscose as a cover yarn. In other words, one or two covers of the first and/or second yarn may be formed of viscose. In certain embodiments, the first as well as the second yarn have one or two viscose covers (cover layers). For example, the first yarn may have a viscose cover, e.g. an inner viscose cover and an outer cover with another yarn. Or an outer viscose cover and an inner cover with another yarn. For example, the second yarn may have one or two viscose covers, e.g. an inner viscose cover and an outer viscose cover. The inner viscose and the outer viscose covers may be the same viscose.

A knitting yarn of the invention can have a core with a density (titer; linear density) of from 20 to 80 dtex, such as from 25 to 70 dtex, from 30 to 65 dtex, from 35 to 60 dtex, or from 40 to 55 dtex. Dtex is the unit for g / 10,000 m. The knitting yarn can have a cover manufactured with a yarn having from 20 to 150 dtex, such as from 40 to 120. As outlined elsewhere herein, a cover yarn can be, in particular, a viscose such as lyocell. In certain embodiments, the viscose (e.g. lyocell) cover has from 50 to 150 dtex and the core has from 35 to 60 dtex.

An inlay yarn of the invention can have a core with a density of from 100 to 600 dtex, such as from 150 to 550 dtex, from 200 to 500 dtex, or from 250 to 450 dtex. The inlay yarn can have one or two covers manufactured with a yarn having from 10 to 60 dtex, such as from 15 to 55 dtex, or from 20 to 50 dtex. In certain embodiments, the one or two viscose covers have from 20 to 50 dtex and the core has from 250 to 450 dtex.

In addition to the knitted fabric, the garment may comprise further components, such as seams connecting different ends of the knitted fabric, e.g., one or more seams in a toe region of the garment; and/or a knitted or woven band forming the proximal end of the garment. The band can be sewn, glued or welded onto the knitted fabric. In preferred embodiments, the knitted fabric is a seamless piece of knitted fabric. This piece may account for 70% or more, preferably 80% or more, of the surface area of the garment, e.g., if the garment is a stocking or arm sleeve. The piece may account for 70% or more, preferably 80% or more, of the surface area of the garment in the area adapted to cover the leg of the wearer, e.g., if the garment is a pair of tights.

The compression garment of the invention can be any type of knitted compression garment. The garment can, for example be selected from the group consisting of a stocking, such as a knee-high stocking, or a thigh-high stocking; a pantyhose (i.e. a pair of tights); or an arm sleeve, such as an arm sleeve with a hand piece (i.e. a section that is adapted to at least partially cover the hand of a wearer) or an arm sleeve without a hand piece. In preferred embodiments, the garment is a stocking or sleeve, most preferably a stocking, such as a knee-high stocking.

It will be understood that the term "compression garment" used herein refers to medical compression garments, i.e. garments which are adapted to provide a compression profile of e.g. any of RAL-GZ 387/1 compression classes I to IV in case the garment is a stocking or pantyhose or RAL-GZ 387/2 I to III in case the garment is an arm sleeve. In certain embodiments, the garment is a class I compression garment according to RAL-GZ 387/1, RAL-GZ 387/2, or RAL-GZ 387/3, and/or a class II compression garment according to Référentiel Technique CBCM ASQUAL.

The compression garment of the invention is suitable for use in the treatment or prevention of various diseases or conditions which require treatment or prevention with compression therapy. These diseases and conditions include diseases and conditions treated with garments according to any of RAL-GZ 387/1 compression classes I to IV - such as class I - in case the garment is a stocking or pantyhose; or RAL-GZ 387/2 I to III - such as class I - in case the garment is an arm sleeve; and/or diseases and conditions treated with garments according to Référentiel Technique CBCM ASQUAL - e.g. class II.

The French standard ASQUAL divides compression garments (orthoses) into four classes, namely class I (13 to 20 hPa, i.e. ≈ 10 to 15 mmHg at the ankle), class II (20.1 to 27 hPa, i.e. 15 to 20 mmHg), class III (27.1 to 48 hPa, i.e. 20 to 36 mmHg) and class IV ( > 48 hPa, i.e. > 36 mmHg).

The German RAL-GZ 387/1 defines that the pressure value at the ankle in its compression classes is 18-21 mmHg (class I), 23-32 mmHg (class II), 34-46 mmHg (class III) and 49 or more mmHg (class IV), respectively. The German RAL-GZ 387/2 defines that the pressure value at the measuring point C₁ (which is located above the wrist bone) is 15-21 mmHg (class I), 23-32 mmHg (class II), and 34-46 mmHg (class III), respectively.

It is defined in the aforementioned standards that it is advantageous to use a garment with a particular compression profile. The garment may, thus, be adapted to apply a pressure according to a predetermined compression profile. The compression profile may provide for a higher pressure at a distal part of a limb and a lower pressure at a proximal part of a limb. In other words, the compression garment of the invention may be adapted to provide pressure according to a predetermined compression profile, e.g. the garment may be adapted to provide a higher pressure at a distal part of a limb and a lower pressure at a proximal part of a limb. Thereby, surplus liquid is encouraged out of the limb and congestion is avoided.

Diseases and conditions treated with garments having class I according to RAL-GZ 387/1 and RAL-GZ 387/2 and/or class II according to Référentiel Technique CBCM ASQUAL can be e.g. selected from the group consisting of phlebologic diseases or conditions; tired, heavy, aching legs; minor varices without edema; mild swelling of the feet, ankles and legs; onset of pregnancy-related varices; primary or secondary lymphedema in International Society of Lymphology (ISL) stages I-III; and lipedema.

The present invention also relates to methods for the treatment or prevention of any of the aforementioned diseases by wearing the compression garment described herein as well as to the compression garment for use in a method of treatment or prevention of any of the aforementioned diseases by wearing the compression garment.

The garment can, e.g., be a compression garment having compression class I, II, III or IV, preferably class I, according to RAL-GZ 387; and/or compression class I, II, III or IV, preferably class II, according to Référentiel Technique CBCM ASQUAL.

In a second aspect, the present invention relates to a method for the manufacture of a compression garment as described herein, comprising the step of knitting the compression garment, preferably on a circular knitting machine. Respective knitting techniques are known in the art and further description of the yarns used is provided elsewhere herein. The knitting can be done by weft-knitting techniques, in particular circular-knitting or flat-knitting, wherein circular-knitting is preferred.

The method may further comprise a step of dyeing the knitted garment at temperatures of 80°C or less, such as 70°C or less. In other words, the dyeing may be carried out at temperatures of from 40°C to 80°C, e.g. 40°C to 70°C.

### BRIEF DESCRIPTION OF FIGURES

Exemplary embodiments of the invention are shown schematically in the drawings.
- **Fig. 1**: schematically shows exemplary compression garments (A: knee-high stocking; B: thigh-high stocking) according to the invention; and
- **Fig. 2**: schematically shows a partial exemplary knitting pattern for a stocking of the invention.

These and other aspects of the invention will be apparent from and elucidated with reference to the embodiments described hereinafter.

### DESCRIPTION OF EMBODIMENTS

Additional advantages, characteristics, and features of the present invention will become clear from the following detailed description of exemplary embodiments with reference to the attached drawings. However, the invention is not restricted to these exemplary embodiments.

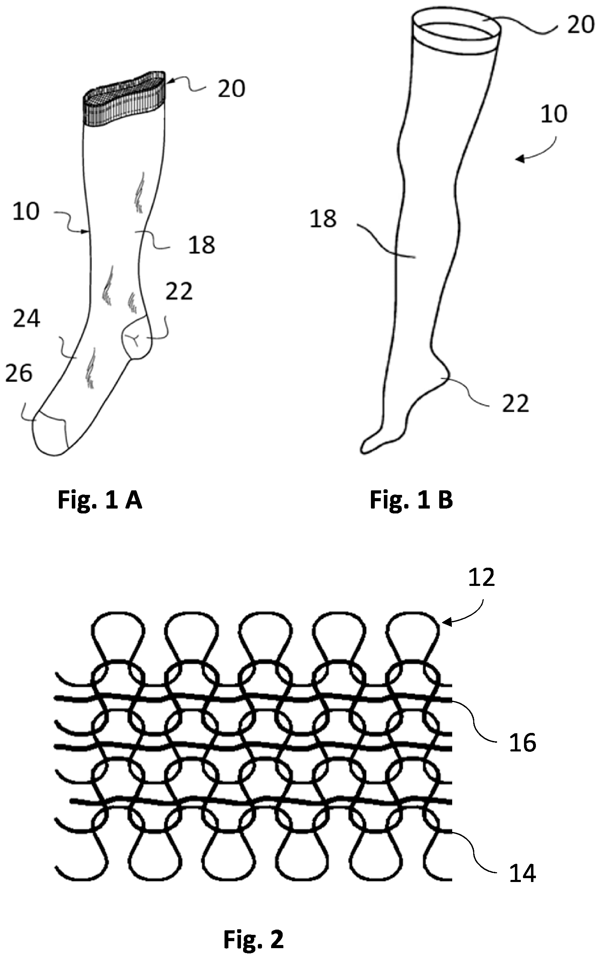

Fig. 1A and Fig. 1B schematically show two different compression garments 10 according to the invention, wherein Fig. 1A shows a knee-high stocking and Fig. 1B shows a thigh-high stocking. Both stockings comprise a band 20 at the proximal end of the garment 10. The band 20 has an anti-slip functionality and improves the fit of the garment 10. The band 20 is, nevertheless, optional in the context of the invention. Alternative garments 10 have no band at their proximal end.

The stockings, further, comprise a tubular section 18 which consists of a knitted fabric 12 as described herein. The tubular section 18 is adapted to cover the calf of the wearer (Fig. 1A) and the calf, knee and thigh of the wearer (Fig. 1B), respectively. In the embodiments shown, the tubular section 18 is seamless. This provides a particularly good fit. For this purpose, the garments 10 are weft-knitted on circular knitting machines.

The garments 10 have a heel portion 22, foot portion 24 and toe portion 26 which are adapted to cover the heel, foot and toes of the wearer, respectively. These portions are knitted with yarns that differ from the sustainable first yarn 14 and second yarn 16 defined herein. Alternatively, the sections may be knitted with the same yarns.

Fig. 2 schematically shows a partial exemplary knitting pattern for a garment 10 of the invention. The knitting pattern is used at least in the knitted fabric 12 forming the tubular section 18 of the garment 10. It can also be used in other parts of the garment 10.

It can be seen that the first yarn 14 is used as knitting (mesh-forming) yarn in every course (knitting row). The first yarn 14 is a double covered core yarn comprising an elastane core and a viscose cover. Furthermore, the second yarn 16 is used as an inlay thread in every course. The second yarn 16 is a double covered core yarn comprising an elastane core and two viscose covers.

Although the present invention has been described in detail with reference to the exemplary embodiments, it is obvious to those skilled in the art that the invention is not restricted to these exemplary embodiments, but rather that modifications can be made in such a way that individual features are omitted or other combinations of the individual features presented are realized, provided that the scope of protection of the appended claims is not exceeded. The present disclosure includes any and all combinations of the individual features presented.

### REFERENCE SIGNS LIST

- 10: garment
- 12: knitted fabric
- 14: first yarn
- 16: second yarn
- 18: tubular section
- 20: band
- 22: heel portion
- 24: foot portion
- 26: toe portion

## Claims

1. Compression garment (10) comprising a knitted fabric (12), wherein the knitted fabric (12) comprises a first yarn (14) comprising a first viscose and a second yarn (16) comprising a second viscose.

2. Compression garment (10) according to claim 1, comprising 40 % by weight or more of viscose yarns, preferably 50 % or more.

3. Compression garment (10) according to any of claims 1 and 2, wherein the first viscose is lyocell.

4. Compression garment (10) according to any of the preceding claims, wherein the first yarn (14) is a knitting yarn.

5. Compression garment (10) according to any of the preceding claims, wherein the second yarn (16) is an inlay yarn.

6. Compression garment (10) according to any of the preceding claims, wherein the garment (10) comprises 20% by weight or more of the first viscose, preferably 30% by weight or more.

7. Compression garment (10) according to any of the preceding claims, wherein the garment (10) comprises 2 % by weight or more of the second viscose, preferably 4% or more.

8. Compression garment (10) according to any of the preceding claims, wherein the first yarn (14) and/or the second yarn (16) is a covered core yarn, such as a double-covered core yarn.

9. Compression garment (10) according to any of the preceding claims, wherein the first yarn (14) and/or the second yarn (16) has an elastane core.

10. Compression garment (10) according to any of the preceding claims, wherein the garment (10) is adapted to apply a pressure according to a predetermined compression profile.

11. Compression garment (10) according to claim 10, wherein the compression profile provides for a higher pressure at a distal part of a limb and a lower pressure at a proximal part of a limb.

12. Compression garment (10) according to any of the preceding claims, wherein the garment is a class I compression garment according to RAL-GZ 387/1, RAL-GZ 387/2, or RAL-GZ 387/3, and/or a class II compression garment according to Référentiel Technique CBCM ASQUAL.

13. Compression garment (10) according to any of the preceding claims, wherein the garment (12) comprises a tubular section (18).

14. Compression garment (10) according to any of the preceding claims, wherein the garment (10) is a stocking or sleeve.

15. Compression garment (10) according to any of the preceding claims, wherein the garment (10) is a circular-knitted garment.
